# EUROPEAN PATENT SPECIFICATION

(11) **EP 2 737 857 B1**
(45) Date of publication and mention of the grant of the patent: **28.03.2018**
(21) Application number: 13195032.1
(22) Date of filing: 29.11.2013
(51) Int. Cl.: A61B 8/08

(54) **Ultrasonic probe apparatus and control method thereof**
Ultraschalldiagnosevorrichtung und Steuerverfahren dafür
Appareil de sonde ultrasonique et son procédé associé

(30) Priority: 29.11.2012 KR 20120137219
(43) Date of publication of application: 04.06.2014
(73) Proprietor: Samsung Electronics Co., Ltd., Gyeonggi-do 443-742 (KR)
(72) Inventor: Shim, Hwan, Yongin-si (KR); Koh, Hyun-woo, Yongin-si (KR); Kim, Seoung-hun, Hwaseong-si (KR); Kim, Young-tae, Seongnam-si (KR)
(74) Representative: Appleyard Lees IP LLP

(56) References cited:
- WO-A2-2006/032058
- WO-A2-2011/138758
- CN-A- 101 750 609
- US-A1- 2003 149 357
- US-A1- 2008 114 249
- US-A1- 2009 043 203
- US-A1- 2009 112 095
- US-A1- 2011 118 604
- XUJIONG YE* ET AL: "3-D Freehand Echocardiography for Automatic Left Ventricle Reconstruction and Analysis Based on Multiple Acoustic Windows", IEEE TRANSACTIONS ON MEDICAL IMAGING, IEEE SERVICE CENTER, PISCATAWAY, NJ, US, vol. 21, no. 9, 1 September 2002 (2002-09-01), XP011076366, ISSN: 0278-0062

## Description

### BACKGROUND

### 1. Field

Apparatuses and methods consistent with exemplary embodiments relate to an ultrasonic probe apparatus that can transmit ultrasonic image data, which are generated by using echo signals reflected in response to transmitting unfocused or defocused ultrasonic signals, to an external display apparatus and a control method thereof.

### 2. Description of the Related Art

An ultrasound is widely used in the medical field to obtain the internal information of an object because of noninvasive and nondestructive characteristics. The ultrasonic diagnostic system is able to provide doctors in real-time with high-resolution images of the object without the need of a surgery, thereby being frequently used in the medical field.

The probe of the ultrasonic apparatus includes a transducer array to transmit ultrasonic signals and receive reflected ultrasonic signals as echo signals. The ultrasonic probe transmits the received echo signals to the external display apparatus as analog signals. However, the ultrasonic probe needs a heavy multi-conductor wire for transmitting analog electrical signals, which unduly increases the weight of the ultrasonic probe. Also, the analog signal contains a great amount of a signal noise, which may result in poor image quality.

Further, in the ultrasonic apparatus, there is a method to generate image data by transmitting and reflecting focused ultrasonic signals and a method to generate image data by transmitting and reflecting unfocused or defocused ultrasonic signals. If a method for generating the focused ultrasonic signals is used, a separate configuration such as a beamformer is needed to generate the focused ultrasonic signals and the frame rate is small, making it difficult to apply in a variety of applications.

Accordingly, there is a need for methods and apparatuses for transmitting digital image data generated by using unfocused or defocused ultrasonic signals to the external display apparatus.

US 2008/0114249 A1 is background art and discloses a transducer array imaging system.

### SUMMARY

Exemplary embodiments may address at least the above problems and/or disadvantages and other disadvantages not described above. Also, the exemplary embodiments are not required to overcome the disadvantages described above, and an exemplary embodiment may not overcome any of the problems described above.

According to the present invention there is provided an apparatus and method as set forth in the appended claims. Other features of the invention will be apparent from the dependent claims. One or more exemplary embodiments provide an ultrasonic probe apparatus that can transmit digital image data generated by using unfocused or defocused ultrasonic signals to an external display apparatus and a control method thereof.

In accordance with an aspect of an exemplary embodiment, there is provided an ultrasonic probe apparatus, which may include an ultrasound transceiver adapted to receive ultrasonic echo signals reflected by transmitting (e.g. unfocused and/or defocused and/or focused) ultrasonic signals having a first frame rate; a converter adapted to convert each of the plurality of ultrasonic echo signals received by the ultrasound transceiver into a digital signal; an image processor adapted to generate a plurality of image data by processing a plurality of digital signals converted through the converter; a combiner/composer adapted to combine/compose the plurality of image data having a first frame rate into a plurality of composite image data to output them in or at a second frame rate; and a transmitter adapted to transmit the plurality of composite image data having the second frame rate to a (e.g. external) display apparatus.

"Frame rate" might be a frequency of reception or generation, or the like, for example a frequency related to image or signal capture or generation.

The image processor may include a Doppler image processor adapted to generate a plurality of Doppler images by performing Doppler processing for each of the plurality of converted digital signals.

The Doppler processing may include at least one of color Doppler processing, B-mode image processing, and spectral Doppler processing.

If image data having different sizes are generated by receiving the echo signals that are the defocused ultrasonic signals reflected and/or changing of a steering angle of the ultrasonic probe apparatus, the image processor is configured to edit the image data having different sizes (which includes shapes and/or areas) into image data having a (e.g. single) predetermined size (which includes a shape and/or area).

The first frame rate may be determined depending on a measuring depth to be measured by using the ultrasonic probe apparatus.

The second frame rate may be determined depending on a frame rate which the transmitter can transmit.

The transmitter may transmit wirelessly the plurality of composite image data to the display apparatus.

The ultrasound transceiver may be detachable from the ultrasonic probe apparatus.

The ultrasonic probe apparatus may include a wireless charger adapted to charge (e.g. provide) power of the ultrasonic probe apparatus by using one of a magnetic induction method and a magnetic resonance method.

The ultrasonic probe apparatus may include a detector adapted to detect a user touch; and/or a power source adapted to apply power to the ultrasonic probe apparatus if user touch is detected through the detector.

According to an aspect of an exemplary embodiment, a control method of an ultrasonic probe apparatus may include receiving a plurality of ultrasonic echo signals reflected by transmitting (e.g. unfocused and/or defocused and/or focused) ultrasonic signals having a first frame rate; converting each of the plurality of received ultrasonic echo signals into a digital signal; generating a plurality of image data by processing the plurality of converted digital signals; composing/combining the plurality of image data having the first frame into a plurality of composite image data to output them in a second frame rate; and transmitting the plurality of composite image data having the second frame rate to a (e.g. external) display apparatus.

The generating a plurality of image data by processing the plurality of converted digital signals may include generating a plurality of Doppler images by performing Doppler processing for each of the plurality of converted digital signals.

The Doppler processing may include at least one of color Doppler processing, B-mode image processing, and spectral Doppler processing.

The generating a plurality of image data by processing the plurality of converted digital signals include, if image data having different sizes are generated by receiving the echo signals that are the defocused ultrasonic signals reflected and/or changing of a steering angle of the ultrasonic probe apparatus, editing the image data having different sizes (which includes shapes and/or areas) into image data having a (e.g. single) predetermined size (which includes a shape and/or area).

The first frame rate may be determined depending on a measuring depth to be measured by using the ultrasonic probe apparatus.

The second frame rate may be determined depending on a frame rate which the transmitter can transmit.

The transmitting the plurality of composite image data having the second frame rate to an external display apparatus may include transmitting wirelessly the plurality of composite image data to the display apparatus.

The control method may include charging (e.g. providing) power of the ultrasonic probe apparatus by using one of a magnetic induction method and a magnetic resonance method.

The control method may include detecting a user touch; and/or applying, if user touch is detected, power to the ultrasonic probe apparatus.

With an ultrasonic probe apparatus according to various exemplary embodiments, image data obtained by the ultrasonic probe apparatus can be applied to various applications, and, because configuration such as a beamformer and a mixer is not needed, a circuit can be easily integrated within the ultrasonic probe apparatus.

Also, because the ultrasonic probe apparatus processes echo signals and transmits image data, an external apparatus needs to only implement relatively simple conversion of a UI and/or a scan, or image data thereof, thereby requiring low computational complexity. Accordingly, the external apparatus may be implemented with various devices such as a personal computer, a smart phone, etc. in addition to a general ultrasonic display apparatus.

In addition, if and when transmitting image data wirelessly, the ease of use of the ultrasonic probe apparatus can be increased, and data distortion caused by analog wired connection can be minimized. Also, no cumbersome cable is required, which cable could be at least a trip hazard in a medical environment.

### BRIEF DESCRIPTION OF THE DRAWINGS

The above and/or other aspects will become more apparent by describing certain exemplary embodiments, with reference to the accompanying drawings, in which:
FIG. 1 is a view illustrating an ultrasonic diagnostic system according to an exemplary embodiment;
FIG. 2 is a block diagram schematically illustrating a configuration of an ultrasonic probe apparatus according to an exemplary embodiment;
FIG. 3 is a block diagram illustrating a configuration of an ultrasonic probe apparatus according to an exemplary embodiment;
FIG. 4 is a view illustrating an ultrasound transceiver that can be detached according to an exemplary embodiment;
FIG. 5 is a view explaining an imaging method according to an exemplary embodiment;
FIG. 6 is a block diagram schematically illustrating a configuration of an external display apparatus according to an exemplary embodiment;
FIG. 7 is a flowchart for explaining a control method of an ultrasonic probe apparatus according to an exemplary embodiment; and
FIG. 8 is a block diagram illustrating a configuration of an ultrasonic probe apparatus according to an exemplary embodiment.

### DETAILED DESCRIPTION OF EXEMPLARY EMBODIMENTS

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail

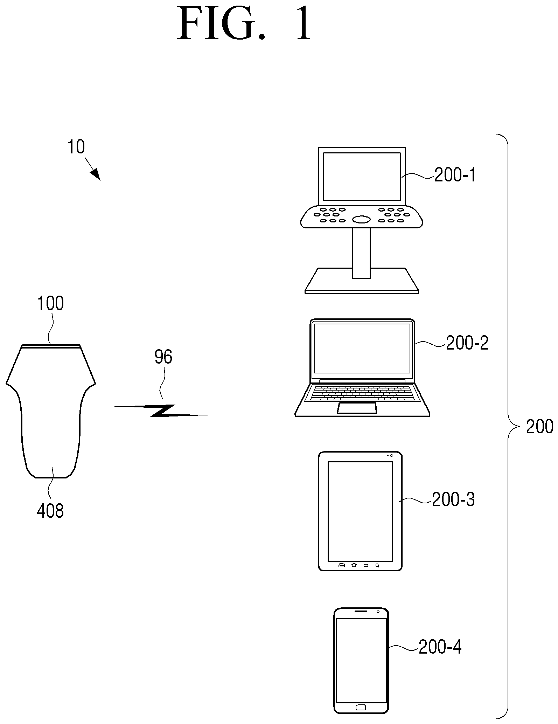

FIG. 1 is a view illustrating an ultrasonic diagnostic system according to an exemplary embodiment. An ultrasonic diagnostic system 10 includes an ultrasonic probe apparatus 100 and a display apparatus 200 which may be physically detached from the main body 408 of the ultrasonic probe apparatus 100.

The ultrasonic probe apparatus 100 generates unfocused or defocused ultrasonic signals, and transmits the generated ultrasonic signals to a diagnostic object (e.g. an object to be diagnosed). For example, the ultrasonic probe apparatus 100 may transmit the ultrasonic signals to generate image data with a first frame rate. For example, the ultrasonic probe apparatus 100 may transmit the ultrasonic signals so that three thousand sheets (e.g. frames) of image data are acquired per second.

The ultrasonic probe apparatus 100 obtains ultrasonic data by receiving echo signals reflected by the diagnostic object, and converts the analog ultrasonic data into digital signals.

The ultrasonic probe apparatus 100 generates a plurality of image data by performing image processes (e.g., demodulation, decimation) for the ultrasonic data converted into digital signals.

The ultrasonic probe apparatus 100 generates a plurality of composite image data by composing or otherwise combining the plurality of image data to have a second frame rate in order to transmit the plurality of image data to the display apparatus 200. For example, if three thousand sheets of image data are generated per second, the ultrasonic probe apparatus 100 may generate a hundred composite image data by composing thirty image data in each composite. For example, the ultrasonic probe apparatus 100 may generate a plurality of composite image data by composing the plurality of image data by considering the resolution of the image data and/or the frame rate with which it can communicate with the display apparatus 200.

The ultrasonic probe apparatus 100 may transmit the generated composite image data to the display apparatus 200. For example, because the ultrasonic probe apparatus 100 transmits the composite image data as the digital data to the display apparatus 200, the image data may be transmitted wirelessly via a network 96. Also, even though the ultrasonic probe apparatus 100 may use wires for transmitting and receiving signals, amount and weight of the wires may be substantially reduced as compared to that of the related art ultrasound apparatus which transmits the image data as analog signals.

The display apparatus 200 outputs audio and/or video signals by processing the composite image data received from the ultrasonic probe apparatus 100. For example, the display apparatus 200 may control the size of the composite image data by considering the resolution of the display apparatus 200.

As illustrated in FIG. 1, the display apparatus 200 according to an exemplary embodiment may be implemented as at least one of an ultrasonic diagnostic system 200-1, a personal computer (PC) 200-2, a tablet computer 200-3, and a smart phone 200-4. However, this is only an exemplary embodiment, and the display apparatus 200 may be implemented with different devices.

The ultrasonic probe apparatus 100 can transmit the composite image data to a single display apparatus 200 or to multiple display apparatuses.

By the ultrasonic diagnostic system as described above, users will be able to receive more easily diagnostic services using ultrasonic waves.

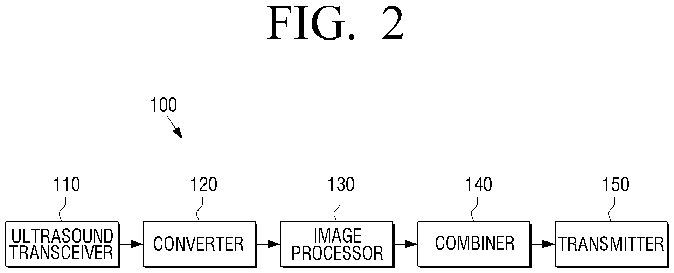

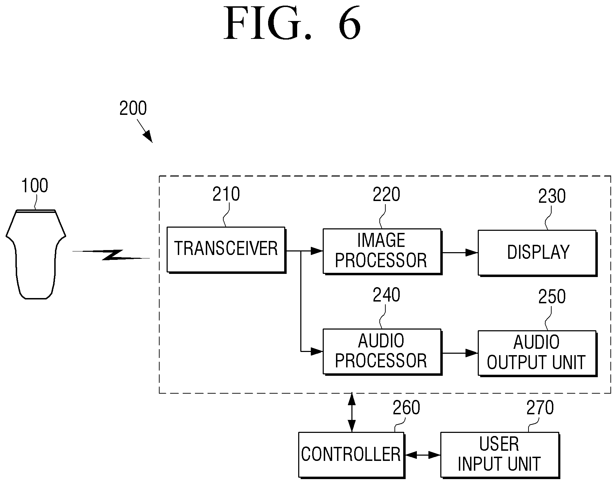

The ultrasonic probe apparatus 100 is described below in detail with reference to FIGS. 2 to 5, and the display apparatus 200 is described below in detail with reference to FIG. 6.

FIG. 2 is a block diagram schematically illustrating a configuration of the ultrasonic probe apparatus 100 according to an exemplary embodiment. As illustrated in FIG. 2, the ultrasonic probe apparatus 100 includes an ultrasound transceiver 110, a converter 120, an image processor 130, a composer/combiner 140, and a transmitter 150.

The ultrasound transceiver 110 generates unfocused or defocused ultrasonic signals, and transmits the generated ultrasonic signals to a diagnostic object (e.g., a human body). For example, the unfocused ultrasonic signal may be a plane wave, and the defocused ultrasonic signal may be a fan-shaped spherical wave.

The ultrasound transceiver 110 obtains ultrasonic data by receiving echo signals reflected by the diagnostic object, in an analog form.

The converter 120 converts the analog ultrasonic data into a digital data. For example, the converter 120 may include an analog to digital converter (ADC).

The image processor 130 obtains image data by image-processing the digital ultrasonic data. In detail, the image processor 130 may perform the image processing of the ultrasonic data by performing at least one of demodulation and decimation, thereby generating the plurality of image data.

If image data having different sizes are generated due to receiving echo signals that are the reflected in response to transmitting the defocused ultrasonic signals and/or changing of a steering angle of the ultrasonic probe apparatus, the image processor 130 may edit the image data having different sizes into image data of a predetermined size. In detail, when the combiner 140 combines the plurality of image data, if the plurality of image data is to take different areas, noise may occur. Therefore, the image processor 130 may edit the plurality of image data so that the plurality of image data becomes images for the same area and output the edited image data to the combiner 140.

The combiner 140 combines the plurality of image data into a plurality of composite image data in order to output the plurality of image data received with a first frame rate in a second frame rate.

In order to increase the resolution of images that will be displayed and to decrease signal-to-noise ratio, the combiner 140 may generate a plurality of composite image data by composing image data more than a predetermined number of frames. For example, the combiner 140 may generate composite image data by composing image data of more than 100 frames.

Also, the combiner 140 may combine the plurality of image data by considering a frame rate by which the ultrasonic probe apparatus 100 can communicate with the display apparatus 200. For example, if the ultrasonic probe apparatus 100 transmits image data of less than 100 frames per second to the display apparatus 200, the ultrasonic probe apparatus 100 may configure and combine the image data so that composite image data have a frame rate of less than 100 frames per second.

The transmitter 150 transmits the composite image data combined by the combiner 140 to the display apparatus 200 via a network 96, by using a wireless communication module such as WiFi, Bluetooth, UWB, WiGig, Zigbee, etc. As another example, the transmitter 150 may transmit the composite image data by using a wired communication module such as IEEE 1394, USB, etc.

By using the ultrasonic probe apparatus 100 as described above, the users may easily receive the portable ultrasonic diagnostic service.

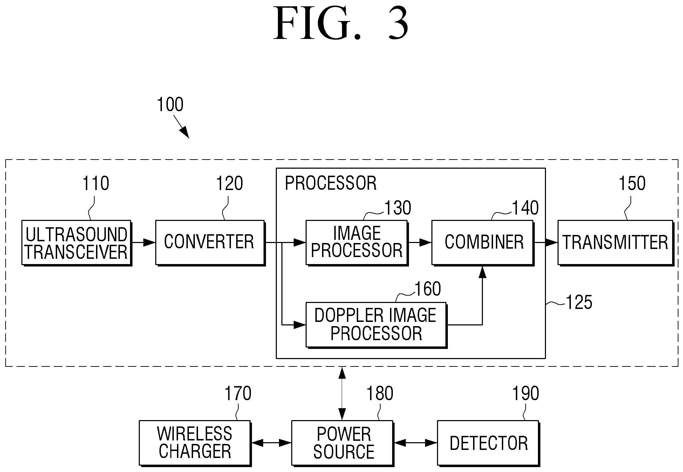

FIG. 3 is a block diagram illustrating a configuration of an ultrasonic probe apparatus 100 according to an exemplary embodiment. As illustrated in FIG. 3, the ultrasonic probe apparatus 100 may include an ultrasound transceiver 110, a converter 120, a processor 125, a transmitter 150, a wireless charger 170, a power source 180, and a detector 190. The processor 125 may include an image processor 130, a combiner 140, and a Doppler image processor 160.

The ultrasound transceiver 110 generates unfocused ultrasonic signals as a plane wave or defocused ultrasonic signals as a spherical wave, transmits the generated ultrasonic signals to the object, and obtains ultrasonic data by receiving echo signals.

For example, the ultrasound transceiver 110 may transmit the ultrasonic signals so that image data having a first frame rate are generated. For example, the first frame rate may be determined depending on a measuring depth h to be measured by using the ultrasonic probe apparatus 100. For example, if the depth to be measured is 15 cm and a velocity of ultrasound in the abdomen of a person is about 1500 m/s, it takes 1/5000 seconds for the ultrasonic signal to be transmitted and reflected and, thus, the ultrasound transceiver 110 may transmit the ultrasonic signals to acquire 5000 sheets of image data per second.

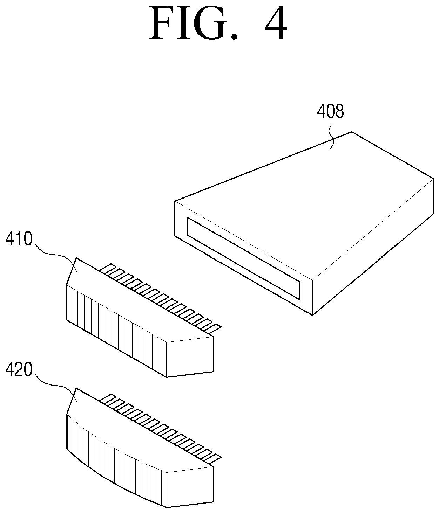

With reference to FIG. 4, the ultrasound transceiver 110 according to an exemplary embodiment may be detachably connected to a main body 408 of the ultrasonic probe apparatus 100 and may include a transducer 410 to generate unfocused ultrasonic signals as a plane wave and a transducer 420 to generate defocused ultrasonic signals as a spherical wave. Because the ultrasound transceiver 110 is detachable, a transducer 410 and a transducer 420 may be fitted alternately into the ultrasonic probe apparatus 100. Accordingly, the user may alternately insert different types of transducers depending on the diagnostic part, i.e., application.

The converter 120 converts the analog ultrasonic data acquired in the ultrasound transceiver 110 into a digital form by using an ADC.

The processor 125 may control overall operation of the ultrasonic probe apparatus 100. Particularly, the processor 125 may control ultrasonic signal transmission timing of the ultrasound transceiver 110 in order to change the first frame rate depending on the measuring depth which the user wants to measure or simply for image capture rates, e.g. in particular for moving objects or changing objects.

Also, the processor 125 may perform various image processing operations in order to transmit the composite image data to the display apparatus 200. Particularly, the processor 125 may include the image processor 130, the combiner 140, and the Doppler image processor 160 for the image processing operation.

The image processor 130 may generate a plurality of image data by image-processing the digital ultrasonic data, as for example, performing the demodulation and /or decimation.

The image processor 130 may edit the digital image data as image data for the same area in order to combine the entered image data. In detail, image data for different areas may be obtained depending on the type and/or steering angle of the ultrasound transceiver 110.

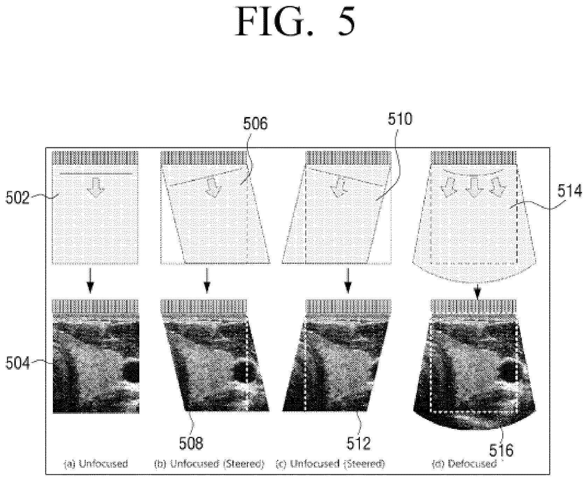

With reference to FIG. 5, if unfocused ultrasonic signals are transmitted and received and the steering angle is perpendicular to the target (reference numeral 502), the image processor 130 may obtain an image datum as illustrated by reference numeral 504. If the unfocused ultrasonic signals are transmitted and received and the steering angle is bent to the right (reference numeral 506), the image processor 130 may obtain an image datum as illustrated by reference numeral 508. If the unfocused ultrasonic signals are transmitted and received and the steering angle is bent to the left (reference numeral 510), the image processor 130 may obtain an image datum as illustrated by reference numeral 512. If defocused ultrasonic signals are transmitted and received (reference numeral 514), the image processor 130 may obtain an image datum as illustrated by reference numeral 516.

However, if the image data for the different areas determined by the type of the ultrasonic signal and the steering angle are combined as obtained, the composite image data may contain a great amount of noise.

Accordingly, in order to combine image data for the same area, the image processor 130 may edit the image data so that only the respective dotted area of FIG. 5 is left and the remaining area is cut off.

On the other hand, in FIG. 5, the image data are edited based on when the steering angle is a right angle, but this is only an exemplary embodiment. The rest of the image data may be edited based on a first image datum for generating a composite image datum. For example, if a first composite image datum is generated by composing from first image datum to 100^{th} image datum and a second composite image datum is generated by composing from 101^{st} image datum to 200^{th} image datum, the image processor 130 may edit from second image datum to the 100^{th} image datum based on the first image datum, and may edit from 102^{nd} image datum to the 200^{th} image datum based on the 101^{st} image datum.

The combiner 140 may generate a plurality of composite image data by composing the plurality of image data generated in the image processor 130.

For example, the combiner 140 may combine the image data by considering the resolution of the composite image data. In detail, if the image data may be generated by using the defocused or unfocused ultrasounds, image data having a lot of frames may be obtained, but the resolution thereof may be lower than that of image data that are generated by using focused ultrasonic signals. Accordingly, the plurality of image data is combined so that the resolution of the image data may be increased and the signal-to-noise ratio thereof may be lowered. Accordingly, the combiner 140 may generate composite image data by composing image data of more than a predetermined number of frames. For example, the combiner 140 may generate the composite image data by composing image data of more than 100 frames. For example, the combiner 140 may determine the number of frames of the image data to be combined according to the user input.

Also, the combiner 140 may combine the image data by considering a frame rate that can be transmitted to the display apparatus 200. For example, if the transmitter 150 can transmit less than 100 frames per second to the display apparatus 200, the combiner 140 may combine the image data so that composite image data have a frame rate of less than 100 frames per second.

For example, the number of composite frames considering the resolution and the frame rate that can be transmitted to the display apparatus 200 may be determined independently. For example, if 5000 sheets of image data can be generated per second and 100 sheets of composite image data per second can be transmitted to the display apparatus 200, the combiner 140 does not need to combine 50 sheets of image data into a single composite image datum. In other words, the combiner 140 may generate 50 sheets of composite image data in a way of composing from first image datum to 50^{th} image datum into a first composite image datum and composing from 51^{st} image datum to 100^{th} image datum into a second composite image datum. However, this is only an exemplary embodiment. Alternatively, the combiner 140 may generate 50 sheets of composite image data in a way of composing from the first image datum to 100^{th} image datum into a first composite image datum and composing from 51^{st} image datum to 150^{th} image datum into a second composite image datum.

As described above, because composite image data having a variety of resolution and frame rates can be generated by composing the image data in various ways, it can be applied to various applications.

The Doppler image processor 160 generates a plurality of Doppler images by performing Doppler processing for each of ultrasonic data of a plurality of digital signals. For example, the Doppler processing may include at least one of color Doppler processing, B-mode image processing, and spectral Doppler processing.

For example, the Doppler image processor 160 may generate dynamic images by using a Doppler filter.

The Doppler image processor 160 may output the processed Doppler image data to the combiner 140 which may compose the Doppler image data to have a predetermined frame, and transmit the image data to the display apparatus 200.

As described above, the Doppler image data are generated, and then the generated Doppler image data may be combined and transmitted to the display apparatus 200. However, this is only an exemplary embodiment. The Doppler image data may be generated by using the composite image data.

The transmitter 150 transmits at least one of the composite image data and the Doppler image data to the display apparatus 200. For example, the transmitter 150 may transmit the composite image data or the Doppler image data in a wired or wireless manner. For example, the transmitter 150 may transmit the composite image data by using a wireless communication module such as WiFi, Bluetooth, UWB, WiGig, Zigbee, etc., and/or by using a wired communication module such as IEEE 1394, USB, etc.

Also, the transmitter 150 may transmit the composite image data or the Doppler image data to an external cloud server or a hospital server as well as the display apparatus 200.

As described above, the combiner 140 generates the plurality of composite image data by composing the plurality of image data generated in the image processor 130. However, this is only an exemplary embodiment. The image processor 130 may generate a plurality of composite image data by directly composing the plurality of image data without configuration of the combiner 140. In other words, the image processor 130 and the combiner 140 may be implemented as a single hardware device.

Also, as described above, the combiner 140 combines the Doppler images outputted from the Doppler image processor 160. However, this is only an exemplary embodiment.

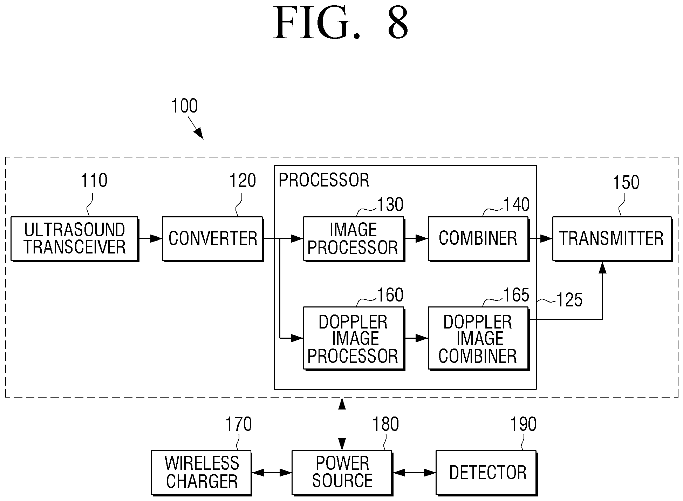

As illustrated in FIG. 8, a separate Doppler image combiner 165 may combine the Doppler images. In detail, the Doppler image processor 160 generates the Doppler images by using the Doppler filter, and then outputs them to the Doppler image combiner 165. The Doppler image combiner 165 may generate the composite Doppler images by composing the Doppler image data to have a predetermined frame, and may output the composite Doppler images to the transmitter 150.

The wireless charger 170 charges (i.e. provides) the power of the ultrasonic probe apparatus 100 by using one of a magnetic induction method and a magnetic resonance method, and supplies the electric power to the power source 180. For example, the magnetic induction method is technology in which a magnetic field generated in the primary coil of a charging pad by causing current to flow through electromagnetic induction is induced to the secondary coil disposed on an object to be charged, thereby supplying current. The magnetic resonance method is technology in that the charging pad and the object to be charged are equipped with resonance coils having the same frequency and the power is sent with the frequency by using the resonance. For example, the wireless charger 170 may include a coil for wireless charging in the main body of the ultrasonic probe apparatus 100.

The power source 180 supplies the electric power to each component of the ultrasonic probe apparatus 100 by using the power charged (i.e. provided) by the wireless charger 170.

The detector 190 detects the user touch. For example, the detector 190 may include a sensor that can detect the user touch, as for example, at least one of an electromagnetic sensor, a gyro sensor, a pressure-sensitive sensor, etc. For example, the power source 180 may supply power to the ultrasonic probe apparatus 100 only if a user touch is detected by the detector 190.

In addition, the ultrasonic probe apparatus 100 may include a motion processor (not illustrated), a voice processor (not illustrated), a self-charger (not illustrated), etc., to increase ease-of-use.

In detail, the motion processor may detect a user's motion and match a detected motion pattern to one of pre-stored motion patterns by comparing a detected motion with the pre-stored motion patterns. The motion processor may perform a function corresponding to the detected and matched motion pattern. For example, when the user shakes the ultrasonic probe apparatus a pre-determined number of times, e.g. 100 multiple times, the motion processor may perform a function corresponding to the shaking motion by detecting the user's motion.

The voice processor may detect a user's voice and search for a voice that matches the recognized voice among the pre-stored voices by comparing the recognized voice with the pre-stored voices. The voice processor may perform a function corresponding to the detected and matched voice. For example, when the user utters a voice command of "power-on," the voice processor may perform a function to turn on the power the ultrasonic probe apparatus 100 by recognizing the user's voice command.

The self-charger may charge or provide the power by using super-capacitors for self-generation in the form of fiber and/or the related generation of static electricity. For example, the super-capacitors may be provided with or be in connection with, or be clothing, gloves, aprons, etc.

By using the ultrasonic probe apparatus 100 as described above, the user will be able to receive the ultrasonic diagnostic service easier.

FIG. 6 is a block diagram schematically illustrating a configuration of a display apparatus 200 according to an exemplary embodiment. As illustrated in FIG. 6, a display apparatus 200 includes a transceiver 210, an image processor 220, a display 230, an audio processor 240, an audio output unit 250, a controller 260, and a user input unit 270.

The transceiver 210 receives the composite image data or the Doppler image data from the ultrasonic probe apparatus 100. Also, the transceiver 210 may transmit setting information (e.g., the resolution of image data, etc.) inputted by the user input unit 270 to the ultrasonic probe apparatus 100.

The image processor 220 may perform signal processing to display the composite image data or the Doppler image data. Particularly, if the resolution of the input composite image data does not match the resolution of the display 230, the image processor 220 may perform a scan conversion function to adjust the size of the composite image data.

The display 230 displays the composite image data or Doppler image data processed in the image processor 220.

The audio processor 240 performs signal processing so that signals can be output through the audio output unit 250 based on the audio data received from the ultrasonic probe apparatus 100. For example, the audio processor 240 may perform the signal processing by using Hilbert transform, etc.

The audio output unit 250 outputs audio data that is signal-processed in the audio processor 240. For example, the audio output unit 250 may include a speaker.

The controller 260 controls overall operation of the display apparatus 200. Particularly, the controller 260 may control the image processor 220 and the display 230 to process and display the composite image data by the user setting inputted through the user input unit 270.

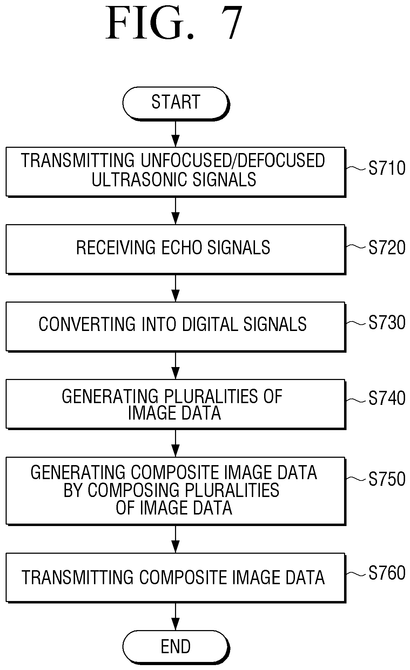

A control method of the ultrasonic probe apparatus 100 is described below in detail with reference to FIG. 7.

The ultrasonic probe apparatus 100 transmits unfocused or defocused (or focused) ultrasonic signals to a diagnostic object (operation S710).

The ultrasonic probe apparatus 100 receives echo signals that are reflected by the diagnostic object (operation S720).

The ultrasonic probe apparatus 100 converts the echo signals from an analog form into digital signals (operation S730).

The ultrasonic probe apparatus 100 generates a plurality of image data by performing signal processing of the digital signals (operation S740).

The ultrasonic probe apparatus 100 generates composite image data by composing the plurality of image data (operation S750).

The ultrasonic probe apparatus 100 transmits the composite image data to the display apparatus 200 (operation S760).

By the control method of the ultrasonic probe apparatus as described above, the user will be able to receive the ultrasonic diagnostic service easier.

Program codes for performing a control method according to various exemplary embodiments as described above may be stored in a non-transitory computer-readable medium. The non-transitory computer-readable medium may be a medium that can store data in a semi-permanent manner and that can be read by devices. In detail, the various applications or programs may be stored in and provided with the non-transitory computer readable medium such as a CD, a DVD, a hard disc, a Blu-ray disc, an USB, a memory card, a ROM, etc.

Attention is directed to all papers and documents which are filed concurrently with or previous to this specification in connection with this application and which are open to public inspection with this specification.

## Claims

1. An ultrasonic probe apparatus (100) comprising:
an ultrasound transmitting/receiving unit (110) adapted to transmit unfocused or defocused ultrasonic signals to an object and receive reflected ultrasonic echo signals having a first frame rate;
a conversion unit (120) adapted to convert the plurality of ultrasonic echo signals received by the ultrasound transmitting/receiving unit into digital signals;
an image processing unit (130) adapted to generate a plurality of image data by processing the digital signals converted by the conversion unit;
a composing unit (140) adapted to compose the plurality of image data having the first frame rate into a plurality of composite image data having a second frame rate; and
a transmitting unit (150) adapted to transmit the plurality of composite image data having the second frame rate to an external display apparatus (200) **characterised in that** the plurality of image data having different sizes and shapes are generated due to receiving the ultrasonic echo signals that are reflected in response to transmitting the defocused ultrasonic signals or transmitting the unfocused ultrasonic signals and changing a steering angle of the ultrasonic probe apparatus, and the image processing unit is adapted to edit the plurality of image data having the different sizes and shapes into the plurality of image data all having the same predetermined size and shape by leaving image data in an area corresponding to the predetermined size and shape, and cutting off the remaining area of the image data.

2. The ultrasonic probe apparatus of claim 1, wherein
the image processing unit comprises a Doppler image processing unit adapted to generate a plurality of Doppler images by performing Doppler processing for each of the digital signals.

3. The ultrasonic probe apparatus of claim 2, wherein
the Doppler processing comprises at least one of color Doppler processing, B-mode image processing, and spectral Doppler processing.

4. The ultrasonic probe apparatus of any of claims 1 to 3, wherein
the first frame rate is determined depending on a measuring depth to be measured by using the ultrasonic probe apparatus.

5. The ultrasonic probe apparatus of any of claims 1 to 4, wherein
the second frame rate is determined depending on a frame rate which the transmitting unit can transmit.

6. The ultrasonic probe apparatus of any of claims 1 to 5, wherein
the transmitting unit transmits the plurality of composite image data wirelessly to the external display apparatus.

7. The ultrasonic probe apparatus of any of claims 1 to 6, wherein
the ultrasound transmitting/receiving unit is detachable from the ultrasonic probe apparatus.

8. The ultrasonic probe apparatus of any of claims 1 to 7 further comprising:
a wireless charging unit adapted to charge power of the ultrasonic probe apparatus by using one of a magnetic induction method and a magnetic resonance method.

9. The ultrasonic probe apparatus of any of claims 1 to 8 further comprising:
a detection unit adapted to detect a user touch; and
a power supply unit adapted to apply power to the ultrasonic probe apparatus when the user touch is detected by the detection unit.

10. A control method of an ultrasonic probe apparatus comprising:
transmitting unfocused or defocused ultrasonic signals to an object (S710);
receiving a plurality of ultrasonic echo signals that are reflected from the object and have a first frame rate (S720);
converting the plurality of received ultrasonic echo signals into digital signals (S730);
generating a plurality of image data by processing the converted digital signals (S740);
composing the plurality of image data having the first frame into a plurality of composite image data having a second frame rate (S750); and
transmitting the plurality of composite image data having the second frame rate to an external display apparatus (S760) wherein
the generating the plurality of image data by processing the digital signals comprises generating the plurality of image data having different sizes and shapes due to receiving the ultrasound echo signals that are reflected in response to transmitting the defocused ultrasonic signals or transmitting the unfocused ultrasonic signals and changing a steering angle of the ultrasonic probe apparatus, and
editing the plurality of image data having the different sizes and shapes into the plurality of image data all having the same predetermined size and shape by leaving image data in an area corresponding to the predetermined size and shape, and cutting off the remaining area of the image data.

11. The control method of claim 10, wherein
the generating the plurality of image data by processing the digital signals comprises generating a plurality of Doppler images by performing Doppler processing for each of the converted digital signals.

12. The control method of claim 11, wherein
the Doppler processing comprises at least one of color Doppler processing, B-mode image processing, and spectral Doppler processing.

13. The control method of any of claims 10 to 12, wherein
the first frame rate is determined depending on a measuring depth to be measured by using the ultrasonic probe apparatus.

## Patentansprüche

1. Ultraschalldiagnosevorrichtung (100), aufweisend:
eine Ultraschallsende-/empfangseinheit (110), die angepasst ist, unfokussierte oder defokussierte Ultraschallsignale an ein Objekt zu senden und reflektierte Ultraschallechosignale, die eine erste Framerate aufweisen, zu empfangen;
eine Umwandlungseinheit (120), die angepasst ist, die mehreren Ultraschallechosignale, die von der Ultraschallsende-/empfangseinheit empfangen werden, in digitale Signale umzuwandeln;
eine Bildverarbeitungseinheit (130), die angepasst ist, mehrere Bilddaten durch Verarbeiten der von der Umwandlungseinheit umgewandelten digitalen Signale zu erzeugen;
eine Zusammenstellungseinheit (140), die angepasst ist, die mehreren Bilddaten, die die erste Framerate aufweisen, zu mehreren zusammengestellten Bilddaten, die eine zweite Framerate aufweisen, zusammenzustellen; und
eine Sendeeinheit (150), die angepasst ist, die mehreren zusammengestellten Bilddaten, die die zweite Framerate aufweisen, an eine externe Anzeigevorrichtung (200) zu senden, **dadurch gekennzeichnet, dass**
die mehreren Bilddaten, die unterschiedliche Größen und Formen aufweisen, erzeugt werden aufgrund Empfangens der Ultraschallechosignale, die als Reaktion auf das Senden der defokussierten Ultraschallsignale oder Senden der unfokussierten Ultraschallsignale reflektiert werden, und Änderns eines Steuerwinkels der Ultraschalldiagnosevorrichtung, und die Bildverarbeitungseinheit angepasst ist, die mehreren Bilddaten, die unterschiedliche Größen und Formen aufweisen, in die mehreren Bilddaten, die alle die gleiche vorbestimmte Größe und Form aufweisen, zu editieren, durch Belassen von Bilddaten in einem Bereich, der der vorbestimmten Größe und Form entspricht, und Abschneiden des verbleibenden Bereichs der Bilddaten.

2. Ultraschalldiagnosevorrichtung nach Anspruch 1, wobei
die Bildverarbeitungseinheit eine Doppler-Bildverarbeitungseinheit aufweist, die angepasst ist, durch Ausführen einer Doppler-Verarbeitung für jedes der digitalen Signale mehrere Doppler-Bilder zu erzeugen.

3. Ultraschalldiagnosevorrichtung nach Anspruch 2, wobei
die Doppler-Verarbeitung mindestens eines von einer Farb-Doppler-Verarbeitung, einer B-Modus-Bildverarbeitung, und einer Spektral-Doppler-Verarbeitung aufweist.

4. Ultraschalldiagnosevorrichtung nach einem der Ansprüche 1 bis 3, wobei
die erste Framerate in Abhängigkeit von einer zu messenden Messtiefe unter Verwendung der Ultraschalldiagnosevorrichtung bestimmt wird.

5. Ultraschalldiagnosevorrichtung nach einem der Ansprüche 1 bis 4, wobei
die zweite Framerate in Abhängigkeit von einer Framerate, die die Sendeeinheit senden kann, bestimmt wird.

6. Ultraschalldiagnosevorrichtung nach einem der Ansprüche 1 bis 5, wobei
die Sendeeinheit die mehreren zusammengestellten Bilddaten drahtlos zu der externen Anzeigevorrichtung sendet.

7. Ultraschalldiagnosevorrichtung nach einem der Ansprüche 1 bis 6, wobei
die Ultraschallsende-/empfangseinheit von der Ultraschalldiagnosevorrichtung abnehmbar ist.

8. Ultraschalldiagnosevorrichtung nach einem der Ansprüche 1 bis 7, ferner aufweisend:
eine drahtlose Ladeeinheit, die angepasst ist, die Leistung der Ultraschalldiagnosevorrichtung unter Verwendung entweder eines magnetischen Induktionsverfahrens oder eines Magnetresonanzverfahrens aufzuladen.

9. Ultraschalldiagnosevorrichtung nach einem der Ansprüche 1 bis 8, ferner aufweisend:
eine Detektionseinheit, die angepasst ist, eine Berührung durch einen Benutzer zu detektieren; und eine Energieversorgungseinheit, die angepasst ist, Energie auf die Ultraschalldiagnosevorrichtung aufzubringen, wenn die Berührung durch einen Benutzer durch die Detektionseinheit detektiert wird.

10. Steuerverfahren für eine Ultraschalldiagnosevorrichtung, aufweisend:
Senden von unfokussierten oder defokussierten Ultraschallsignalen an ein Objekt (S710);
Empfangen von mehreren Ultraschallechosignalen, die von dem Objekt reflektiert werden und eine erste Framerate aufweisen (S720);
Umwandeln der mehreren empfangenen Ultraschallechosignale in digitale Signale (S730);
Erzeugen von mehreren Bilddaten durch Verarbeiten der umgewandelten digitalen Signale (S740);
Zusammenstellen der mehreren Bilddaten, die den ersten Frame haben, in mehrere zusammengestellte Bilddaten, die die zweite Framerate haben (S750); und
Senden der mehreren zusammengestellten Bilddaten, die die zweite Framerate haben, an eine externe Anzeigevorrichtung (S760), wobei
das Erzeugen der mehreren Bilddaten durch Verarbeiten der digitalen Signale Erzeugen der mehreren Bilddaten aufweist, die unterschiedliche Größen und Formen aufweisen, aufgrund Empfangens der Ultraschallechosignale, die in Reaktion auf das Senden der defokussierten Ultraschallsignale oder Senden der unfokussierten Ultraschallsignale reflektiert werden, und Änderns eines Steuerwinkels der Ultraschalldiagnosevorrichtung, und
Editieren der mehreren Bilddaten, die unterschiedliche Größen und Formen aufweisen, in die mehreren Bilddaten, die alle die gleiche vorbestimmte Größe und Form aufweisen, durch Belassen von Bilddaten in einem Bereich, der der vorbestimmten Größe und Form entspricht, und Abschneiden des verbleibenden Bereichs der Bilddaten.

11. Steuerverfahren nach Anspruch 10, wobei
das Erzeugen der mehreren Bilddaten durch Verarbeiten der digitalen Signale Erzeugen von mehreren Doppler-Bildern durch Ausführen einer Doppler-Verarbeitung für jedes der umgewandelten digitalen Signale aufweist.

12. Steuerverfahren nach Anspruch 11, wobei
die Doppler-Verarbeitung mindestens eines von einer Farb-Doppler-Verarbeitung, B-Modus-Bildverarbeitung, und Spektral-Doppler-Verarbeitung aufweist.

13. Steuerverfahren nach einem der Ansprüche 10 bis 12, wobei
die erste Framerate in Abhängigkeit von einer zu messenden Messtiefe unter Verwendung der Ultraschalldiagnosevorrichtung bestimmt wird.

## Revendications

1. Appareil à sonde ultrasonique (100), comprenant :
une unité de transmission/de réception d'ultrasons (110), adaptée pour transmettre des signaux ultrasoniques non focalisés ou défocalisés à un objet et recevoir des signaux d'écho ultrasoniques réfléchis présentant une première cadence de trame ;
une unité de conversion (120), adaptée pour convertir la pluralité de signaux d'écho ultrasoniques reçus par l'unité de transmission/de réception d'ultrasons en signaux numériques ;
une unité de traitement d'image (130), adaptée pour générer une pluralité de données d'image en traitant les signaux numériques convertis par l'unité de conversion ;
une unité de composition (140), adaptée pour composer la pluralité de données d'image présentant la première cadence de trame en une pluralité de données d'image composite présentant une seconde cadence de trame ; et
une unité de transmission (150), adaptée pour transmettre la pluralité de données d'image composite présentant la seconde cadence de trame à un appareil d'affichage externe (200),
**caractérisé en ce que**
la pluralité de données d'image présentant des tailles et formes différentes sont générées en raison de la réception des signaux d'écho ultrasoniques qui sont réfléchis en réponse à la transmission des signaux ultrasoniques défocalisés ou la transmission des signaux ultrasoniques non focalisés et du changement d'un angle d'orientation de l'appareil à sonde ultrasonique, et l'unité de traitement d'image est adaptée pour éditer la pluralité de données d'image présentant les tailles et formes différentes en la pluralité de données d'image présentant toutes les mêmes taille et forme prédéterminées en laissant des données d'image dans une zone correspondant aux taille et forme prédéterminées, et sectionnant la zone restante des données d'image.

2. Appareil à sonde ultrasonique selon la revendication 1, dans lequel
l'unité de traitement d'image comprend une unité de traitement d'image Doppler adaptée pour générer une pluralité d'images Doppler en effectuant un traitement Doppler pour chacun des signaux numériques.

3. Appareil à sonde ultrasonique selon la revendication 2, dans lequel
le traitement Doppler comprend au moins l'un de : traitement Doppler de couleur, traitement d'image mode B, et traitement Doppler spectral.

4. Appareil à sonde ultrasonique selon l'une quelconque des revendications 1 à 3, dans lequel
la première cadence de trame est déterminée en fonction d'une profondeur de mesure destinée à être mesurée en utilisant l'appareil à sonde ultrasonique.

5. Appareil à sonde ultrasonique selon l'une quelconque des revendications 1 à 4, dans lequel
la seconde cadence de trame est déterminée en fonction d'une cadence de trame que l'unité de transmission peut transmettre.

6. Appareil à sonde ultrasonique selon l'une quelconque des revendications 1 à 5, dans lequel
l'unité de transmission transmet la pluralité de données d'image composite sans fil à l'appareil d'affichage externe.

7. Appareil à sonde ultrasonique selon l'une quelconque des revendications 1 à 6, dans lequel
l'unité de transmission/de réception d'ultrasons est détachable de l'appareil à sonde ultrasonique.

8. Appareil à sonde ultrasonique selon l'une quelconque des revendications 1 à 7 comprenant en outre :
une unité de charge sans fil adaptée pour réaliser la charge électrique de l'appareil à sonde ultrasonique en utilisant l'un de : un procédé d'induction magnétique et un procédé de résonance magnétique.

9. Appareil à sonde ultrasonique selon l'une quelconque des revendications 1 à 8, comprenant en outre :
une unité de détection adaptée pour détecter un toucher d'utilisateur ; et
une unité d'alimentation électrique adaptée pour appliquer de l'énergie électrique sur l'appareil à sonde ultrasonique lorsque le toucher d'utilisateur est détecté par l'unité de détection.

10. Procédé de commande d'un appareil à sonde ultrasonique comprenant :
la transmission de signaux ultrasoniques non focalisés ou défocalisés à un objet (S710) ;
la réception d'une pluralité de signaux d'écho ultrasoniques qui sont réfléchis à partir de l'objet et présentent une première cadence de trame (S720) ;
la conversion de la pluralité de reçu signaux d'écho ultrasoniques en signaux numériques (S730) ;
la génération d'une pluralité de données d'image en traitant les signaux numériques convertis (S740) ;
la composition de la pluralité de données d'image présentant la première trame en une pluralité de données d'image composite présentant une seconde cadence de trame (S750) ; et
la transmission de la pluralité de données d'image composite, présentant la seconde cadence de trame, à un appareil d'affichage externe (S760) dans lequel
la génération de la pluralité de données d'image en traitant les signaux numériques comprend la génération de la pluralité de données d'image présentant des tailles et formes différentes en raison de la réception des signaux d'écho ultrasoniques qui sont réfléchis en réponse à la transmission des signaux ultrasoniques défocalisés ou la transmission des signaux ultrasoniques non focalisés et du changement d'un angle d'orientation de l'appareil à sonde ultrasonique, et
l'édition de la pluralité de données d'image présentant les tailles et formes différentes en la pluralité de données d'image présentant toutes les mêmes taille et forme prédéterminées en laissant des données d'image dans une zone correspondant aux taille et forme prédéterminées, et sectionnant la zone restante des données d'image.

11. Procédé de commande selon la revendication 10, dans lequel
la génération de la pluralité de données d'image en traitant les signaux numériques comprend la génération d'une pluralité d'images Doppler en effectuant un traitement Doppler pour chacun des signaux numériques convertis.

12. Procédé de commande selon la revendication 11, dans lequel
le traitement Doppler comprend au moins l'un de : traitement Doppler de couleur, traitement d'image mode B, et traitement Doppler spectral.

13. Procédé de commande selon l'une quelconque des revendications 10 à 12, dans lequel
la première cadence de trame est déterminée en fonction d'une profondeur de mesure destinée à être mesurée en utilisant l'appareil à sonde ultrasonique.
